# EUROPEAN PATENT SPECIFICATION

(11) **EP 3 821 188 B1**
(45) Date of publication and mention of the grant of the patent: **30.08.2023**
(21) Application number: 19835159.5
(22) Date of filing: 10.07.2019
(51) Int. Cl.: F25D 17/04, B01D 53/34, A61L 9/20

(54) **REFRIGERATOR APPLIANCES AND ACTIVE AIR FILTER ASSEMBLIES**
KÜHLSCHRANKGERÄTE UND AKTIVE LUFTFILTERANORDNUNGEN
APPAREILS FRIGORIFIQUES MÉNAGERS ET ENSEMBLES FILTRE À AIR ACTIF

(30) Priority: 11.07.2018 US 201816032148
(43) Date of publication of application: 19.05.2021
(73) Proprietor: Qingdao Haier Refrigerator Co., Ltd., Laoshan District Qingdao Shandong 266101 (CN); Haier Smart Home Co., Ltd., Qingdao, Shandong 266101 (CN); Haier US Appliance Solutions, Inc., Wilmington, DE 19801 (US)
(72) Inventor: PFAFFINGER, Robert Charles, Jr., Louisville, KY 40299 (US); PRINCE, Ryan John, Frankfort, IL 60423 (US)
(74) Representative: Ziebig Hengelhaupt Intellectual Property Attorneys Patentanwaltsanwaltskanzlei PartGmbB
(86) International application number: PCT/CN2019/095456
(87) International publication number: WO 2020/011207

(56) References cited:
- WO-A1-2017/055951
- WO-A1-2017/161530
- CA-A1- 3 033 069
- CN-A- 101 099 911
- CN-A- 102 741 633
- CN-A- 104 204 695
- CN-A- 104 457 128
- CN-A- 106 016 521
- DE-A1-102016 216 961
- JP-A- H01 219 481
- KR-A- 20060 080 075
- US-A1- 2016 341 467

## Description

### FIELD OF THE INVENTION

The present subject matter relates generally to refrigerator appliances, and more particularly to active air filter assemblies that may be utilized with refrigerator appliances.

### BACKGROUND OF THE INVENTION

Refrigerator appliances generally include a cabinet that defines a chilled chamber for receipt of food items for storage. For example, the cabinet can define a fresh food chamber and, may further define a freezer chamber. The fresh food chamber can be maintained at a temperature greater than the freezing point of water. Conversely, the freezer chamber can be maintained at a temperature equal to or less than the freezing point of water.

In some instances, refrigerator appliances include air filters as part of a filter assembly for filtering the air within the fresh food chamber to remove odors, etc. However, in many cases, it can be difficult for a user to know if the filter is properly installed. Moreover, in some cases, it can be difficult for a user to know if (or what portions of) a filter assembly should be deactivated when a filter is being replaced. As an example, if a fan is included near the filter, rotation of the fan may create difficulties for a user trying to install a filter. The fan may interfere with placement of the filter or risk striking the filter as it moves. As another example, if a lighting assembly is provided (e.g., in order to provide light as a catalyst for certain types of filter media), light emitted from the lighting assembly may create difficulties for a user. In particular, the user may be exposed to excessive amounts of light or have difficulty seeing various portions of the filter assembly.

DE 10 2016 216961 A1 teaches a refrigeration unit comprising a storage chamber (3) for refrigerated goods and an air line (5) extending from the chamber (3), on which an odor filter (10) and an odor sensor (14) are arranged, wherein the filter (10) and the odor sensor (14) are arranged in succession. The air line (5) comprises a branch, which bypasses the odor filter (10), and connects the odor sensor (14) with the storage chamber (3).

CA 3 033 069 A1 discloses devices, systems and methods for obtaining data representative of the condition of an air filter media of an air filter, and for using such data to present an indication of the air filter media condition to a user.

WO 2017/161530 A1 discloses a room air purifier comprising a housing (9), an air filter (100), a RFID reader (143), a RFID tag (120), and a Wireless communication unit (142). Furthermore, a method on how to operating the room air purifier is taught.

Accordingly, improved air filters for refrigerator appliances are desired in the art. In particular, improved active air filters addressing one or more of the above identified issues would be advantageous.

### BRIEF DESCRIPTION OF THE INVENTION

The claimed subject-matter is defined by the independent claims 1 and 7. Further preferred embodiments are defined by the dependent claims 2 through 6 and 8 through 13. Aspects and advantages of the invention will be set forth in part in the following description, or may be obvious from the description, or may be learned through practice of the invention.

These and other features, aspects and advantages of the present invention will become better understood with reference to the following description and appended claims. The accompanying drawings, which are incorporated in and constitute a part of this specification, illustrate embodiments of the invention and, together with the description, serve to explain the principles of the invention.

### BRIEF DESCRIPTION OF THE DRAWINGS

A full and enabling disclosure of the present invention, including the best mode thereof, directed to one of ordinary skill in the art, is set forth in the specification, which makes reference to the appended figures.
FIG. 1 provides a front, perspective view of a refrigerator appliance according to exemplary embodiments of the present disclosure.
FIG. 2 provides a front, perspective view of the exemplary refrigerator appliance of FIG. 1 with doors of the refrigerator appliance shown in open positions to reveal a fresh food chamber of the refrigerator appliance.
FIG. 3 provides a schematic view of an air filter assembly according to exemplary embodiments of the present disclosure.
FIG. 4 provides a side, schematic view of an air filter assembly according to exemplary embodiments of the present disclosure.
FIG. 5 provides a side, schematic view of an air filter assembly according to exemplary embodiments of the present disclosure.
FIG. 6 provides an exploded, perspective view of an air filter assembly according to exemplary embodiments of the present disclosure.
FIG. 7 provides a front, perspective view of an air filter assembly according to exemplary embodiments of the present disclosure.
FIG. 8 provides a flow chart illustrating a method of operating a refrigerator appliance according to exemplary embodiments of the present disclosure.

### DETAILED DESCRIPTION OF THE INVENTION

Reference now will be made in detail to embodiments of the invention, one or more examples of which are illustrated in the drawings. Each example is provided by way of explanation of the invention, not limitation of the invention. In fact, it will be apparent to those skilled in the art that various modifications and variations can be made in the present invention without departing from the scope or spirit of the invention. For instance, features illustrated or described as part of one embodiment can be used with another embodiment to yield a still further embodiment. Thus, it is intended that the present invention covers such modifications and variations as come within the scope of the appended claims and their equivalents.

It is noted that, for the purposes of the present disclosure, the terms "includes" and "including" are intended to be inclusive in a manner similar to the term "comprising." Similarly, the term "or" is generally intended to be inclusive (i.e., "A or B" is intended to mean "A or B or both"). The terms "first," "second," and "third" may be used interchangeably to distinguish one component from another and are not intended to signify location or importance of the individual components.

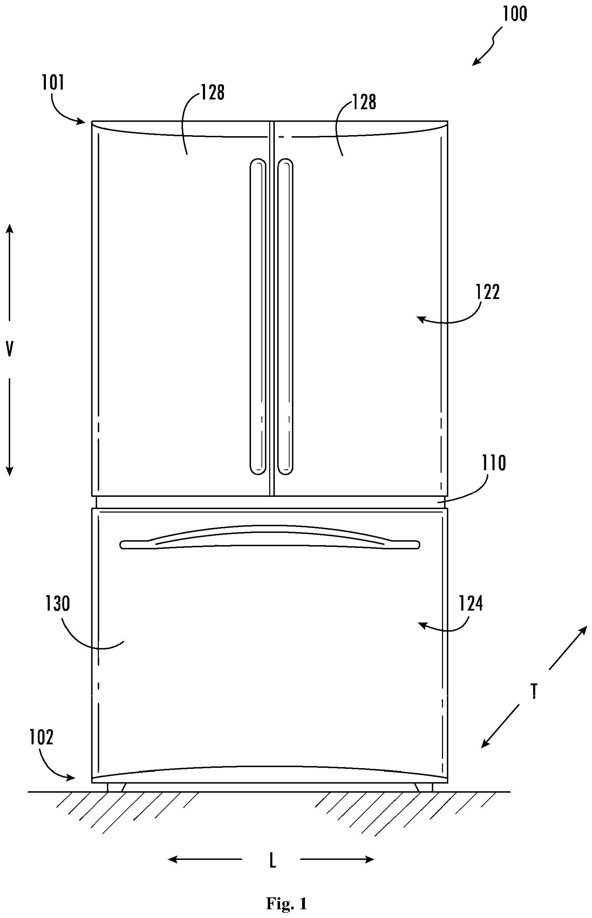

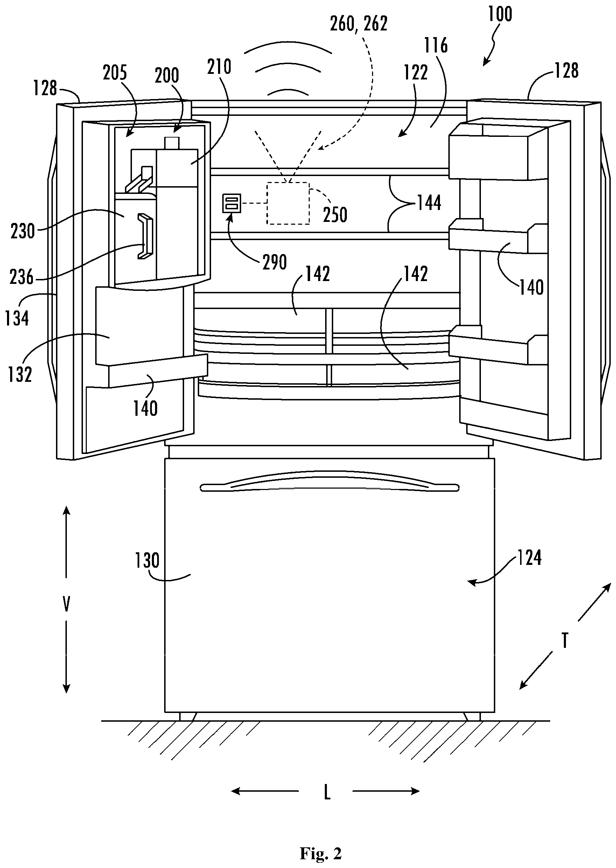

Turning now to the figures, FIG. 1 provides a front, perspective view of a refrigerator appliance 100 according to exemplary embodiments of the present disclosure with refrigerator doors 128 of the refrigerator appliance 100 shown in a closed position. FIG. 2 provides a front, perspective view of refrigerator appliance 100 with refrigerator doors 128 shown in an open position to reveal a fresh food chamber 122 of the refrigerator appliance 100. In some embodiments, appliance 100 includes an ice making assembly 200. In exemplary embodiments as shown, the ice making assembly 200 can be positioned generally within or adjacent to a fresh food chamber 122 of refrigerator appliance 100 when the doors 128 are in closed positions. Alternatively, however, the ice making assembly 200 can be positioned generally within or adjacent to a freezer chamber 124 of refrigerator appliance 100 when the doors 128 are in closed positions.

Refrigerator appliance 100 includes a cabinet or housing 110 that extends between a top portion 101 and a bottom portion 102 along a vertical direction V. Cabinet 110 defines one or more chilled chambers for receipt of food items for storage. In particular, as shown, cabinet 110 defines a fresh food chamber 122 positioned at or adjacent top portion 101 of cabinet 110 and a freezer chamber 124 arranged at or adjacent bottom portion 102 of cabinet 110. Fresh food chamber 122 is thus disposed above freezer chamber 124 along the vertical direction V in these embodiments. As such, refrigerator appliance 100 is generally referred to as a bottom mount refrigerator appliance. It is recognized, however, that the benefits of the present disclosure apply to other types and styles of refrigerator appliances such as, for example, a top mount refrigerator appliance or a side-by-side style refrigerator appliance. Consequently, the description set forth herein is for illustrative purposes only and is not intended to be limiting in any aspect to any particular refrigerator chamber configuration.

In exemplary embodiments, cabinet 110 includes a first sidewall 112 and a second sidewall (not shown) that are generally spaced apart along a lateral direction L. Further, cabinet 110 may include a rear wall 116, which is generally spaced apart from refrigerator door(s) 128 and freezer door(s) 130 of the refrigerator appliance 100 along a transverse direction T. The vertical, lateral, and transverse directions V, L, T may each be perpendicular to each other. Sidewalls 112 and rear wall 116 of cabinet 110 may define the fresh food chamber 122 and freezer chamber 124.

One or more refrigerator doors 128 are rotatably mounted or hinged to an edge of cabinet 110 for selectively accessing fresh food chamber 122. Each door 128 may include an inner surface 132 and an outer surface 134, between which the door 128 is generally defined. In addition, one or more freezer doors 130 are arranged below refrigerator doors 128 for selectively accessing freezer chamber 124. Freezer door 130 may be coupled to a freezer drawer (not shown) slidably mounted within freezer chamber 124. As discussed above, refrigerator doors 128 and freezer door 130 are shown in the closed position in FIG. 1, and refrigerator doors 128 are shown in the open position in FIG. 2.

Turning especially to FIG. 2, various storage components are mounted within fresh food chamber 122 to facilitate storage of food items therein. In certain embodiments, the storage components include drawers 142 and racks 144 that are mounted within fresh food chamber 122. Bins 140 may additionally be provided, such as mounted on doors 128, and may be disposed within fresh food chamber 122 when the doors 128 are in the closed position. Bins 140, drawers 142, and racks 144 are configured for receipt of food items (e.g., beverages or solid food items) and may assist with organizing such food items. As an example, drawers 142 can receive fresh food items (e.g., vegetables, fruits, or cheeses) and increase the useful life of such fresh food items.

As may be seen in FIG. 2, optional embodiments include an ice making assembly 200. Ice making assembly 200 may be disposed within the fresh food chamber 122, the freezer chamber 124, or a door 128, 130. In exemplary embodiments, ice making assembly 200 may be disposed within a door 128. Thus, ice-making assembly 200 can be positioned within fresh-food chamber 122 (e.g., when refrigerator doors 128 are closed). Ice-making assembly 200 is configured for producing ice, as is generally understood.

In embodiments wherein ice-making assembly 200 is disposed within a door 128 (e.g., as shown) ice-making assembly 200 generally includes an ice box 205, which may be a general area defined in one of the doors 128. Various components of the ice-making assembly 200, such as an ice maker 210 and a container 230, may be disposed within the ice box 205. As is understood, ice maker 210 is configured for producing ice. As an example, ice maker 210 can be a nugget or auger style ice maker. Ice produced by ice maker 210 may be provided to and contained in container 230 until use by a consumer. In optional embodiments, container 230 includes, for example, a handle 236 for easy of removal by a consumer. In additional or alternative embodiments, refrigerator appliance 100 includes a suitable apparatus for on demand dispensing of ice from container 230 through, for example, an ice chute (not shown).

Refrigerator appliance 100 generally includes a controller (e.g., main controller 250). In some embodiments, the main controller 250 is configured to control refrigerator appliance 100 operations, as is generally understood. For example, various inputs, sensors and displays (e.g., temperature inputs, temperature sensors, etc.-not shown) may be in communication with the main controller 250. Additionally, the refrigeration cycle apparatus may be in communication with the main controller 250. The main controller 250 may be configured to receive signals from such components and transmit signals to such components to control refrigerator appliance 100 operations.

In certain embodiments, the main controller 250 includes a memory and microprocessor, such as a general or special purpose microprocessor operable to execute programming instructions or micro-control code associated with operation of refrigeration appliance 100. The memory may represent random access memory such as DRAM, or read only memory such as ROM or FLASH. In one embodiment, the processor executes programming instructions stored in memory. The memory may be a separate component from the processor or may be included onboard within the processor. Alternatively, the main controller 250 may be constructed without using a microprocessor (e.g., using a combination of discrete analog or digital logic circuitry; such as switches, amplifiers, integrators, comparators, flip-flops, AND gates, and the like) to perform control functionality instead of relying upon software. Various components as discussed above and as discussed below may be in communication with the main controller 250 via one or more signal lines or shared communication busses.

In optional embodiments, a wireless transmission device 260 is in communication with the main controller 250. The wireless transmission device 260 may be operable to wirelessly transmit data signals. Wireless transmission utilizes electromagnetic or acoustic waves to transmit data signals through atmospheric space rather than along a wire. Wireless transmission device 260 may, for example, be a component of a router, as is generally understood, which is in communication with main controller 250. A wireless receiver 262 may additionally be included in communication with the main controller 250, and may, for example, be integrated with wireless transmission device 260 or be a component of a router.

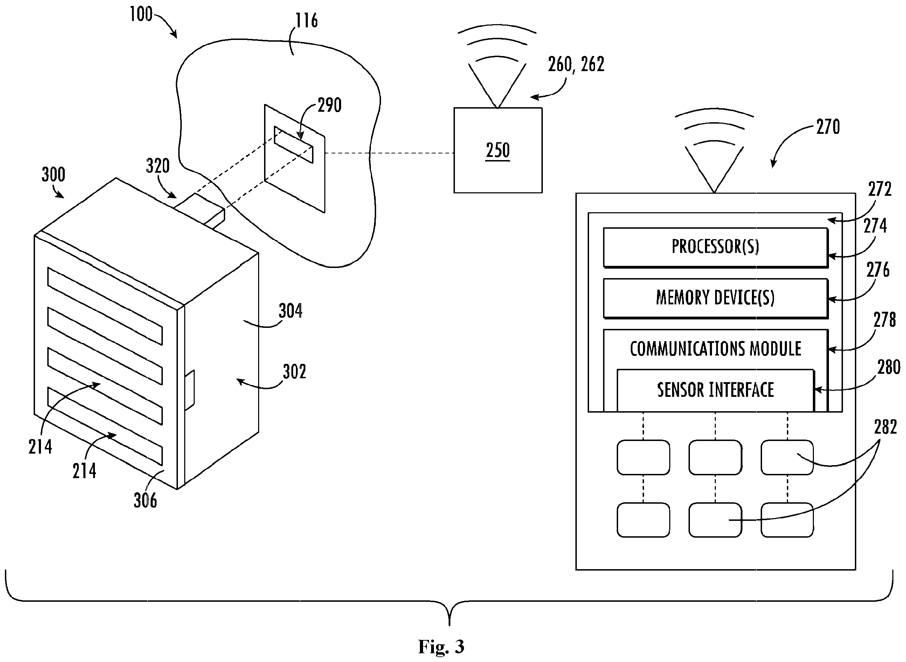

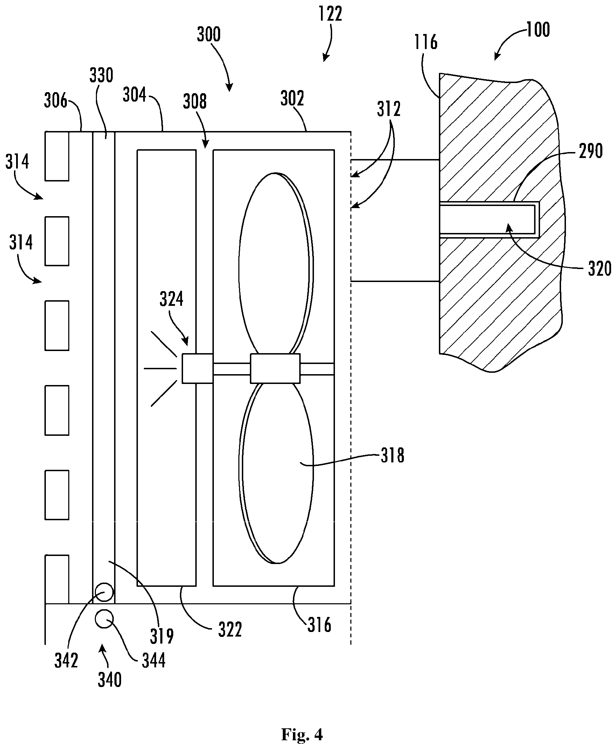

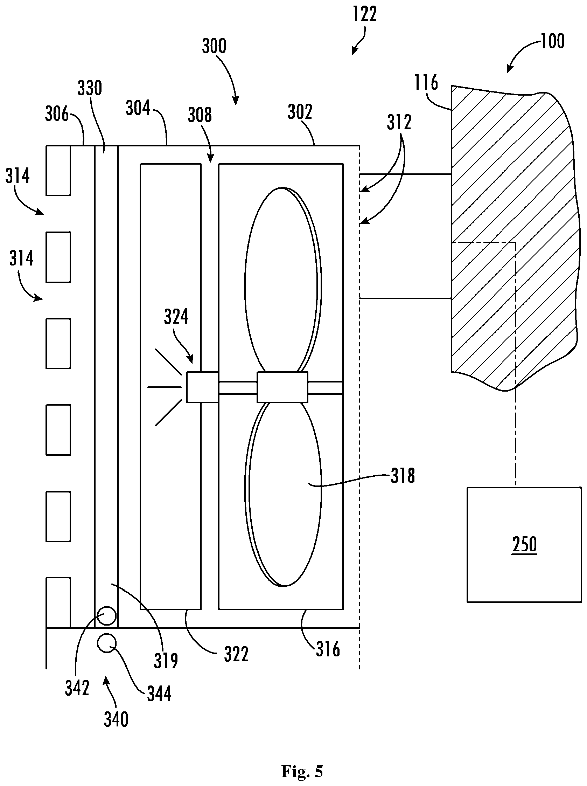

Turning now to FIGS. 3 through 5, various schematic views of an exemplary air filter assembly 300 mounted (e.g., fixedly mounted or, alternatively, removably mounted) within refrigerator appliance 100 are provided. As an example, such as that illustrated in FIGS. 3 and 4, air filter assembly 300 may be removably mounted within a chilled chamber (e.g., fresh food chamber 122) of refrigerator appliance 100. As another example, such as that illustrated in FIG. 5, air filter assembly 300 may be fixedly mounted to a wall (e.g., rear wall 116) within a chilled chamber (e.g., fresh food chamber 122) of refrigerator appliance 100.

As illustrated, main controller 250 may be in communication with a user interface 270 (e.g., wireless communication or, alternatively, wired communication). In general, the user interface 270 may provide the user with access to refrigerator appliance 100 information, such as temperature settings and performance indicators, and may be operable (e.g., as by the user) to modify various settings of the air filter assembly 300 or the refrigerator appliance 100. In optional embodiments, the user interface 270 is independent from refrigerator appliance 100 and may be, for example, a computer (e.g., a desktop computer or a laptop), a tablet, a personal telephone (e.g., smartphone), or an independent device which functions solely to operate and communicate with the refrigerator appliance 100. In alternative embodiments, user interface 270 is included as part of the refrigerator appliance 100 and may be, for example, a region on cabinet 110 or door 128 (FIG. 2) that includes one or more user inputs (e.g., buttons, knobs, dials, etc.) or screens (e.g., LCD or LED displays), as is understood by one of ordinary skill.

In exemplary embodiments, user interface 270 includes a controller (e.g., secondary controller 272). The controller 272 may include one or more processor(s) 274 and associated memory device(s) 276 configured to perform a variety of computer-implemented functions (e.g., of air filter assembly 300 or refrigerator 100). Additionally or alternatively, the controller 272 may also include a communications module 278 to facilitate communications between the user interface 270 and the main controller 250. For instance, the communications module 278 may serve as a wireless interface to permit the controller 272 to transmit or receive refrigerator appliance information. Moreover, the communications module 278 may include an interface 280 (e.g., one or more analog-to-digital converters) to permit input signals to be converted into signals that can be understood and processed by the processor 274. The interface 280 may include or be in communication with input selectors 282 of the user interface 270, through which a user may provide various inputs are desired. In additional or alternative embodiments, the secondary controller is included as, or as part of, main controller 250.

In optional embodiments, such as those illustrated in FIGS. 3 and 4, filter assembly 300 is removably attached to and within refrigerator appliance 100. A connector port 290 may be included in refrigerator appliance 100. A corresponding connector plug 320 may be included with air filter assembly 300.

As shown, connector port 290 may be accessible within the fresh food chamber 122, such as through the rear wall 116 (as shown) or the first sidewall 112 (FIG. 2) or second sidewall. Connector port 290 may be in communication with the main controller 250, and may be configured to provide data communication with the main controller 250 or electrical power. In turn, connector plug 320 may be connectable to the connector port 290 to receive the data communication with the main controller 250 or the electrical power provided by the connector port 290. In exemplary embodiments, the connector port 290 may provide both data communication with the main controller 250 and electrical power. The data communication may, for example, be serial communication. The connector plug 320 may receive both data communication with the main controller 250 and electrical power. Additionally or alternatively, another suitable energy source, such as batteries or another electric connection, may be provided. In exemplary embodiments, the connector port 290 is a universal serial bus ("USB") port while the connector plug 320 may is a universal serial bus ("USB") plug.

In alternative embodiments, such as those illustrated in FIG. 5, filter assembly 300 is fixedly attached within refrigerator appliance 100. One or more suitable adhesives or mechanical fasteners (not pictured) may hold filter assembly 300 to a wall (e.g., rear wall 116) defining a chilled chamber, such as fresh food chamber 122 of refrigerator appliance 100. A wired connection (e.g., is formed by one or more suitable conductive wires or buses) may be established through the wall between air filter assembly 300 and main controller 250 or user interface 270. As is understood, data communication or electrical power may be provided to the wired connection. Additionally or alternatively, another suitable energy source, such as batteries or another electric connection, may be provided.

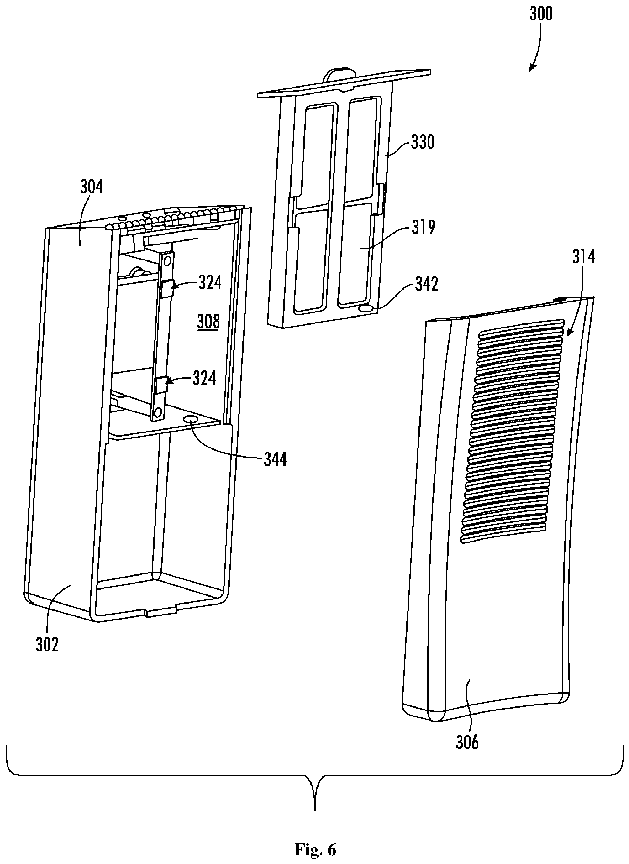

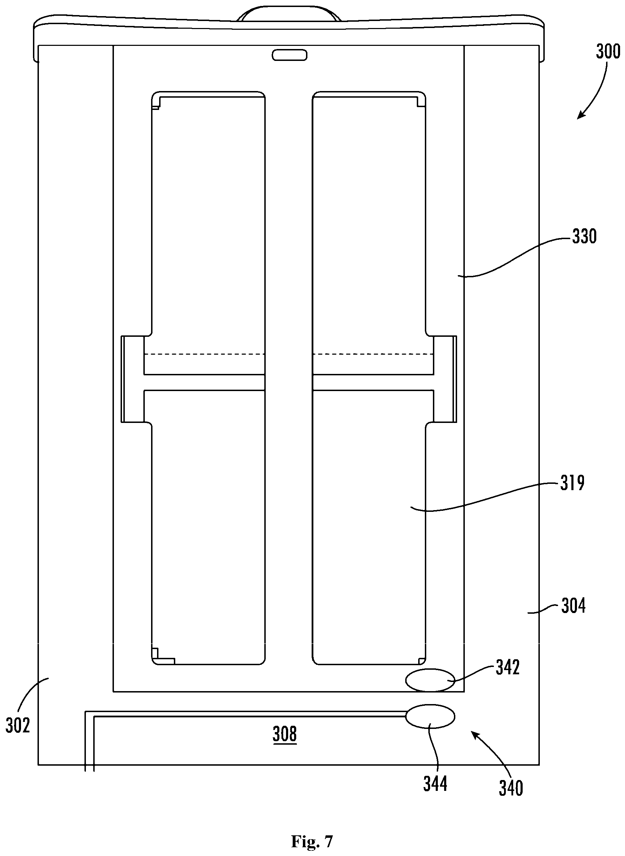

Turning now generally to FIGS. 3 through 7, FIGS. 6 and 7 include various views of an air filter assembly 300 in isolation and according to exemplary embodiments of the present disclosure. Advantageously, air filter assemblies 300 in accordance with the present disclosure are active air filter assemblies 300, which actively flow air therethrough for filtering. For example, as discussed herein, an air filter assembly 300 includes a fan 316, rotation of which flows air through the air filter assembly 300. Air filter assemblies 300 in accordance with the present disclosure may advantageously be relatively inexpensive, reliable, and long lasting.

Generally, air filter assembly 300 includes a housing 302 that may, for example, include a body 304 and a discrete door 306 for accessing an interior 308 of the body 304 (the interior 308 being defined by the housing 302). Various other components of assembly 300 as discussed herein may be included within interior 308. As is understood, door 306 may be pivotable, removable, or otherwise movable between open and closed positions.

One or more inlet apertures 312 and one or more outlet apertures 314 are defined in the housing 302, such as in the body 304 or door 306. For example, inlet apertures 312 may be defined in the body 304. Additionally or alternatively, outlet apertures 314 may be defined in the door 306. Air may be actively flowed from an exterior region (e.g., within the refrigerator appliance 100) to the housing 302 through the inlet apertures 312 into the interior 308, and actively flowed from the interior 308 through the outlet apertures 314 to a region exterior to the housing 302 (e.g., the fresh food chamber 122).

According to the invention, the air filter assembly 300 includes a fan 316 disposed within the housing 302, such as in the interior 308. Fan 316 may, for example, include blades 318 rotatably connected to a motor (not shown). The fan 316 is operable to actively flow air through the inlet apertures 312 and outlet apertures 314, as discussed. For example, blades 318 may be rotatable about a central axis to generate air flow within the interior 308, thus actively flowing air through the inlet apertures 312 and outlet apertures 314.

As shown, a filter medium 319 may be selectively positioned to filter air flowed through the housing 302. In particular, filter medium 319 may be removably positioned within the housing 302 such that a user can readily install and subsequently remove the filter medium 319 from the interior 308. In some such embodiments, a medium frame 330 is fixed to the filter medium 319 to move therewith (e.g., relative to the housing 302). The medium frame 330 may be formed of a relatively solid nonpermeable material (e.g. a suitable cardboard, metal, polymer etc.) and generally supports the filter medium 319. For instance, the medium frame 330 may bound or substantially surround a perimeter of the filter medium 319 and provide rigidity or support to the filter medium 319. When assembled, the medium frame 330 may thus facilitate installation or movement of the filter medium 319 relative to the housing 302.

When installed or otherwise positioned within the housing 302, filter medium 319 may be disposed within the interior 308 between inlet apertures 312 and outlet apertures 314. Air flowing between inlet apertures 312 and outlet apertures 314 may thus pass through filter medium 319 and be filtered to removed particulates, etc., as is generally understood.

Generally, any suitable material(s) or structures may be utilized for filter medium 319. For example, filter medium 319 may be a carbon filter formed from a carbon material, which in exemplary embodiments may be an active carbon material. Additionally or alternatively, a suitable woven or non-woven fabric, molecular sieve, or other suitable structure / material may be utilized. In some embodiments, filter medium 319 includes a catalyst (e.g., photo-catalyst) coated thereon. The catalyst may be activated to kill bacteria, pollutants, etc. Thus, filter medium 319 may include or be provided as a photo-catalyst filtration media. The catalyst may, for example, be activated by light waves (e.g., ultraviolet or visible light waves defined along the color spectrum, as is understood). In exemplary embodiments, for example, a catalyst formed from TiO2 may be utilized.

In certain embodiments, one or more light sources 324 are mounted within housing 302. Generally, the light source(s) 324 may be provided as any suitable light-generating source, such as a light emitting diode (LED), fluorescent bulb, etc. In some embodiments, the light source 324 may include or be provided as an ultraviolet light, which is configured to emit ultraviolet lightwaves. The light source 324 may be disposed within the housing 302, such as in the interior 308. In exemplary embodiments, the light source 324 may be oriented to emit ultraviolet lightwaves towards the filter medium 319. For example, as discussed, filter medium 319 may include a catalyst which is activated by ultraviolet lightwaves. Actuation of the light source 324 may emit lightwaves towards the filter medium 319, which may advantageously activate the catalyst and, for example, cause photocatalytic oxidation.

Optionally, air filter assembly 300 may further include a process control block ("PCB") microprocessor 322. The PCB microprocessor 322 may generally be configured to control operations of the air filter assembly 300, some of which are discussed herein. Further, in exemplary embodiments, the PCB microprocessor 322 is configured for data communication with the controller 250, and may thus transmit or receive data with the controller 250. As an example, such as that illustrated in FIG. 4, PCB microprocessor 322 may be in communication with the connector plug 320 such that data communication is received by PCB microprocessor 322 from controller 250 through connector port 290 and connector plug 320 when connector plug 320 is connected to connector port 290. As another example, air filter assembly 300 may be free of any separate PCB microprocessor. In some such embodiments, the controller 250 is generally configured to control operations of air filter assembly 300.

In exemplary embodiments, ultraviolet light source 324 is in communication with and actuatable by the PCB microprocessor 322 or main controller 250. Accordingly, PCB microprocessor 322 or main controller 250 may selectively actuate the ultraviolet light source 324 to an on position wherein ultraviolet lightwaves are emitted or an off position wherein ultraviolet lightwaves are not emitted. Such selective actuation may, for example, be based on a user selected or factory-determined time period, or be based on other suitable inputs from the PCB microprocessor 322 or main controller 250. For example, in some embodiments, the light source 324 may be actuated on for a suitable time period after a door 128 is opened and then closed. This time period may be adjusted, either manually by a user or automatically by PCB microprocessor 322 or main controller 250, based for example, on the frequency with which door 128 is opened and shut or the length of time that door 128 is left open.

In some embodiments, air filter assembly 300 may further include a field assembly 340 to detect the position of the filter medium 319 or the medium frame 330 (e.g., when the filter medium 319 is mounted within the interior 308). In particular, the field assembly 340 may include a separate field tag 342 and field sensor 344. As illustrated, the field tag 342 is fixed to the filter medium 319. The field sensor 344 is attached to the housing 302 to detect the presence of the field tag 342 (e.g., within the interior 308). According to the invention, the field sensor 344 is in operable communication (e.g., electrical or wireless communication) with the PCB 322 or controller 250.

As a fixed member, the field tag 342 may generally move with the filter medium 319. Thus, when the filter medium 319 or medium frame 330 is removed from the housing 302, the field tag 342 will be similarly removed. In other words, the field tag 342 is movable relative to (and removable from) the housing 302. In some embodiments, the field tag 342 is mounted or otherwise positioned within the filter medium 319 (e.g., radially inward from the medium frame 330). For instance, the field tag 342 may be embedded within the catalyst media. In other embodiments, the field tag 342 positioned on the medium frame 330 itself. The field tag 342 may be spaced apart from the filter medium 319 while still being fixed thereto.

While the field tag 342 may generally move relative to the housing 302, the field sensor 344 may be stationary relative to the housing 302. In particular, the field sensor 344 may be mounted on a suitable location of the housing 302. As an example, the field sensor 344 may be mounted on or within the body 302. As another example, the field sensor 344 may be mounted on or within the door 306. As yet another example, the field sensor 344 may be mounted within at least a portion of the interior 308. The stationary positioning may permit field sensor 344 to detect if or when the field tag 342 is in close proximity, such as when filter medium 319 is correctly positioned within the housing 302.

As is understood, the field tag 342 and the field sensor 344 may be provided as any suitable structure for detecting a visually imperceptible (e.g., electrical or magnetic) field communicated between the field tag 342 in the field sensor 344.

In the invention, the field assembly 340 detects a magnetic field. The field sensor 344 is or includes a magnetic field sensor. The field tag 342 is or includes a permanent magnet. As described above, the field tag 342 (including the permanent magnet) is fixed to the filter medium 319. Moreover, the permanent magnet may be in selective communication with the magnetic field sensor. For instance, placing the filter medium 319 in the correct position within the housing 302 may permit communication between the permanent magnet and the magnetic field sensor. The magnetic field generated by the permanent magnet may be detected by the magnetic field sensor. By contrast, removing filter medium 319 from the housing 302-or otherwise placing the filter medium 319 in a position other than the correct mounted positioned within the housing 302-may prevent or halt communication between the permanent magnet and the magnetic field sensor.

In other embodiments, the field assembly 340 detects a radiofrequency (RF) field. The field sensor 344 may be or include a radio frequency identification (RFID) sensor. The field tag 342 may be or include a radio frequency identification (RFID) tag. As described above, the field tag 342 (including the RFID tag) may be fixed to the filter medium 319. Moreover, the RFID tag may be in selective communication with the RFID reader. In particular, the RFID reader may be configured for receiving a signal from RFID tag.

In some such exemplary embodiments, RFID tag is a passive RFID tag. Thus, RFID reader can receive a radio signal from RFID tag in response to a query or request signal from RFID reader. In particular, RFID tag can generate or transmit the response radio signal utilizing energy transmitted (e.g., wirelessly) to RFID tag from RFID reader via the query or request signal from RFID reader. Thus, RFID tag need not include a battery or other power source in order to generate or transmit the response radio signal. In other exemplary embodiments, RFID tag is an active RFID tag and includes a battery or is connected to a suitable power source. Thus, RFID tag can continuously or intermittently generate or transmit a signal that RFID reader can receive. As will be understood, RFID reader and RFID tag can have any other suitable setup or configuration for placing RFID reader and RFID tag in selective signal communication with each other.

As noted above, the field assembly 340 may generally detect a field communicated between the field tag 342 in the field sensor 344. When the filter medium 319 is correctly positioned within the housing 302, such as in the interior 308, the field sensor 344 may detect a field at the field tag 342. In turn, a detection signal may be transmitted from the field sensor 344 to the PCB 322 or controller 250. The PCB 322 or controller 250 may prompt or enter a first mode. If the detection signal is halted or is not received at the PCB 322 or controller 250, the PCB 322 or controller 250 may prompt or enter a second mode. Moreover, the PCB 322 or controller 250 may direct operations of the refrigerator 100 or filter assembly 300 based on receiving the detection signal (or absence thereof).

In exemplary embodiments, a field assembly 340 is in communication with the PCB microprocessor 322 or main controller 250 (e.g., through field sensor 344). The PCB microprocessor 322 or main controller 250 may be configured to control activation of the light source(s) 324 according to the detected mode. For instance, when the filter assembly 300 is in the first mode, the light sources 324 may be activated (e.g., continuously or along a predetermined pattern) to emit or direct light to the filter medium 319 (i.e., illuminate the filter medium 319). By contrast, when the filter assembly 300 is in the second mode, light sources 324 may be deactivated or otherwise prevented from directing light therefrom (e.g., toward the fresh food chamber 122 or door 306). Advantageously, a user may be protected from potentially damaging lightwaves when the filter medium 319 is not present or properly positioned to block such lightwaves.

In additional or alternative embodiments, PCB microprocessor 322 or main controller 250 may be configured to track the time during which the switch 326 has been continuously actuated to the first mode, thus tracking the time during which the filter medium 319 has been installed (e.g., for filter life and replacement purposes). Filter life or replacement recommendations can then be transmitted to, for example, a user display of the refrigerator appliance 100 or user interface 270.

Notably, user interface 270 may be utilized to interact with various components of the air filter assembly 300. For example, as discussed, user interface 270 can receive data related to the filter medium 326, or the light source 324. Further, user interface 270 may receive data related to the fan 316, which may for example indicate whether the fan 316 is actuated on or off. A user may additionally be able to transmit data signals to air filter assembly 300 to control operation of air filter assembly 300. For example, a user may be able to actuate the fan 316, set a timer for the fan 316, actuate the light source 324, set a timer for the light source 324, reset the timer that tracks switch actuation times 326, etc.

Optionally, the PCB microprocessor 322 or controller 250 may control operation of the air filter assembly 300 and associate such air filter assembly 300 operation with operation of the refrigerator appliance 100. For example, in some embodiments, the air filter assembly 300 and various components thereof may only be operated when a door 128 is opened, or may only be operated when main refrigerator appliance 100 fans are operating. In some embodiments, air filter assembly 300 (such as the fan 316) may operate cooperatively with the main refrigerator appliance 100 fans, such that the fan 316 and main fans alternate operation or operate at different speeds when simultaneously operating. Further, in some embodiments the air filter assembly 300 and various components thereof may only be operated when the refrigerator appliance 100 is in a particular mode, such as a main cooling mode or non-defrost mode. In some embodiments, the air filter assembly 300 may only be operated when, for example, drawers 142 proximate the air filter assembly 300 are opened. In some embodiments, the air filter assembly 300 (such as the fan 316) may be operated at a particular predetermined slower speed during particular times, and at a particular predetermined higher speed during other particular times. For example, a slower speed may be utilized during times of day when users are likely to be in proximity to the refrigerator appliance 100 (i.e., at home, during meal times, etc.) and a higher speed may be utilized during times of day when users are not likely to be in proximity to the refrigerator appliance 100 (i.e., nighttime). If a user interface 270 is utilized and locating features are enabled, these locating features could be utilized to determine whether a user is within a predetermined distance from the refrigerator appliance 100. A slower speed could be utilized when the user is within the predetermined distance, and a higher speed could be utilized when the user is without the predetermined distance. Additionally or alternatively, microprocessor 322 or main controller 250 may include suitable software for modulating power to the light source 324 in order to stay below predefined power ratings for the refrigerator appliance 100.

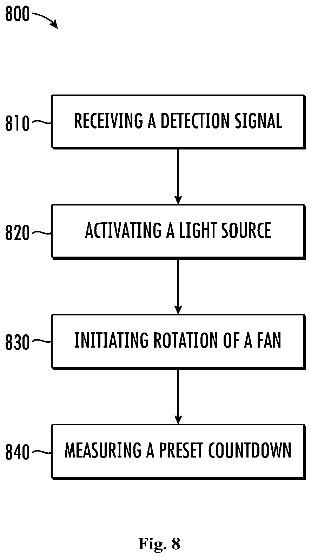

Turning to FIG. 8, a flow chart is provided of a method 800 according to exemplary embodiments of the present disclosure. Generally, the method 800 provides for methods (e.g., filtration operations) of operating a refrigerator appliance 100 or filter assembly 300, as described above. The method 800 can be performed, for instance by the microprocessor 322 or main controller 250. For example, the microprocessor 322 or main controller 250 may be operably coupled to the light sources 324 or fan 316. During operations, the microprocessor 322 or main controller 250 may send signals to and receive signals from the light sources 324 or fan 316. The microprocessor 322 or main controller 250 may further be operably coupled to other suitable components of the refrigerator appliance 100 or filter assembly 300 to facilitate operations generally.

Referring to FIG. 8, at 810, the method 800 includes receiving a detection signal. In particular, the detection signal may be received from the field sensor. As described above, the detection signal may indicate a specific field (e.g., magnetic or radiofrequency field) has been detected between the field sensor and the field tag. In other words, the detection signal may be received following positioning or installation of the filter medium within the housing of the air filter assembly.

In response to 810, the method 800 may include directing air filtration to the air filter assembly. Such air filtration may include one or more of 820, 830, or 840.

At 820, the method 800 includes activating a light source. As discussed above, the light source may illuminate a catalyst within the filter medium. Thus, air passing through the filter medium may be suitably treated. If the detection signal is no longer received (or removal of the filter medium from the housing is otherwise detected), the light source may be deactivated. Notably, activation of light source may be restricted to instances or conditions in which the filter medium is correctly positioned within the housing of the air filter assembly.

At 830, the method 800 includes initiating rotation of the fan. As discussed above, rotation of the fan may motivate an airflow across or through the filter medium. For instance, air may be flowed from the inlet apertures of the filter assembly, through the interior, and subsequently to a chilled chamber of the refrigerator appliance through the outlet apertures of the filter assembly.

At 840, the method 800 includes measuring a preset countdown (e.g., sub-portion of time). In some such embodiments, 840 begins immediately following receipt of the detection signal at 810. The preset countdown may generally correspond to a known lifespan for the filter medium. Expiration of the preset countdown may thus indicate replacement of the filter medium is appropriate. Additionally or alternatively, expiration of the preset countdown may initiate transmission of an alert signal to the user interface or another suitable auditory or visual alert system.

## Claims

1. A refrigerator appliance (100), comprising:
a cabinet (110) defining a chilled chamber (122);
a door (128) for accessing the chilled chamber (122);
an active filter assembly (300) mounted within the cabinet (110) in fluid communication with the chilled chamber (122),
the active filter assembly (300) comprising a housing (302), a filter medium (319) removably positioned within the housing (302),
**characterized by** the active filter assembly (300) further comprising a field tag (342) fixed to the filter medium (319), and a field sensor (344) attached to the housing (302) to detect the field tag (342); and
a controller (250) in operable communication with active filter assembly (300), the controller (250) being configured to initiate a filtration operation, the filtration operation comprising
receiving a detection signal from the field sensor (344) based on detection of the filter medium (319) within the housing (302), and
directing air filtration through the active air assembly in response to receiving the detection signal, wherein the field sensor (344) comprises a magnetic field sensor (344), and wherein the field tag (342) comprises a permanent magnet fixed to the filter medium (319) in selective communication with the magnetic field sensor (344).

2. The refrigerator appliance (100) of claim 1, wherein the filter medium (319) comprises a photo-catalyst filtration media, wherein
the active filter assembly (300) further comprises a light source (324) mounted within the housing (302), wherein
the light source (324) is directed toward the chilled chamber (122).

3. The refrigerator appliance (100) of claim 1, wherein the field tag (342) is positioned within the filtration medium.

4. The refrigerator appliance (100) of claim 1, wherein the active filter assembly (300) further comprises a medium frame (330) fixed to the filter medium (319) in support thereof, and wherein the field tag (342) is positioned on the medium frame (330).

5. The refrigerator appliance (100) of claim 1, wherein the field sensor (344) comprises a radio frequency identification (RFID) sensor, and wherein the field tag (342) comprises a radio frequency identification (RFID) tag fixed to the filter medium (319) in selective communication with the RFID sensor.

6. The refrigerator appliance (100) of claim 5, wherein the RFID tag is a passive RFID tag.

7. An active filter assembly (300) comprising:
a housing (302);
an inlet aperture defined in the housing (302);
an outlet aperture defined in the housing (302);
a filter medium (319) removably positioned within the housing (302) in fluid communication between the inlet aperture and the outlet aperture;
a field tag (342) fixed to the filter medium (319);
a field sensor (344) attached to the housing (302) to detect the field tag (342);
a fan (316) disposed within the housing (302) and operable to actively flow air through the inlet aperture and outlet aperture; and
a controller (250) in operable communication with the field sensor (344), the controller (250) being configured to initiate a filtration operation, the filtration operation comprising
receiving a detection signal from the field sensor (344) based on detection of the filter medium (319) within the housing (302), and
directing air filtration through the active air assembly in response to receiving the detection signal, directing air filtration comprising selectively rotating the fan (316), wherein the field sensor (344) comprises a magnetic field sensor (344), and wherein the field tag (342) comprises a permanent magnet fixed to the filter medium (319) in selective communication with the magnetic field sensor (344).

8. The active filter assembly (300) of claim 7, wherein the filter medium (319) comprises a photo-catalyst filtration media, wherein
the active filter assembly (300) further comprises a light source (324) mounted within the housing (302).

9. The active filter assembly (300) of claim 8, further comprising a door (128) attached to the housing (302), wherein the light source (324) is directed toward the door (128).

10. The active filter assembly (300) of claim 7, wherein the field tag (342) is positioned within the filtration medium.

11. The active filter assembly (300) of claim 7, wherein the active filter assembly (300) further comprises a medium frame (330) fixed to the filter medium (319) in support thereof, and wherein the field tag (342) is positioned on the medium frame (330).

12. The active filter assembly (300) of claim 7, wherein the field sensor (344) comprises a radio frequency identification (RFID) sensor, and wherein the field tag (342) comprises a radio frequency identification (RFID) tag fixed to the filter medium (319) in selective communication with the RFID sensor.

13. The active filter assembly (300) of claim 12, wherein the RFID tag is a passive RFID tag.

## Patentansprüche

1. Kühlschrankgerät (100), umfassend:
einen Schrank (110), der eine gekühlte Kammer (122) definiert;
eine Tür (128) zum Zugang zu der gekühlten Kammer (122);
eine Aktivfilter-Baugruppe (300), die innerhalb des Schranks (110) montiert ist und in Fluid-Verbindung mit der gekühlten Kammer (122) steht,
wobei die Aktivfilter-Baugruppe (300) ein Gehäuse (302) und ein Filtermedium (319) umfasst, das entfernbar innerhalb des Gehäuses (302) positioniert ist,
**dadurch gekennzeichnet, dass** die Aktivfilter-Baugruppe (300) ferner ein Feld-Etikett (342), das an dem Filtermedium (319) fest angebracht ist, und einen Feldsensor (344) umfasst, der an dem Gehäuse (302) angebracht ist, um das Feld-Etikett (342) zu erkennen; und
eine Steuereinheit (250) in betriebsfähiger Kommunikation mit der Aktivfilter-Baugruppe (300), wobei die Steuereinheit (250) dazu eingerichtet ist, einen Filtriervorgang einzuleiten, wobei der Filtriervorgang umfasst:
Empfangen eines Erkennungssignals von dem Feldsensor (344) basierend auf der Erkennung des Filtermediums (319) innerhalb des Gehäuses (302) und
Führen einer Luftfilterung durch die aktive Luft-Baugruppe als Reaktion auf das Empfangen des Erkennungssignals, wobei der Feldsensor (344) einen Magnetfeldsensor (344) umfasst und wobei das Feld-Etikett (342) einen an dem Filtermedium (319) fest angebrachten Dauermagnet in selektiver Kommunikation mit dem Magnetfeldsensor (344) umfasst.

2. Kühlschrankgerät (100) nach Anspruch 1, wobei das Filtermedium (319) ein Fotokatalysator-Filtermedium umfasst, wobei
die Aktivfilter-Baugruppe (300) ferner eine Lichtquelle (324) umfasst, die innerhalb des Gehäuses (302) montiert ist, wobei
die Lichtquelle (324) auf die gekühlte Kammer (122) gerichtet ist.

3. Kühlschrankgerät (100) nach Anspruch 1, wobei das Feld-Etikett (342) innerhalb des Filtermediums positioniert ist.

4. Kühlschrankgerät (100) nach Anspruch 1, wobei die Aktivfilter-Baugruppe (300) ferner einen Mediumrahmen (330) umfasst, der zur Stützung des Filtermediums (319) fest an diesem angebracht ist, und wobei das Feld-Etikett (342) auf dem Mediumrahmen (330) positioniert ist.

5. Kühlschrankgerät (100) nach Anspruch 1, wobei der Feldsensor (344) einen Radiofrequenzidentifikation-Sensor (RFID-Sensor) umfasst und wobei das Feld-Etikett (342) ein an dem Filtermedium (319) fest angebrachtes Radiofrequenzidentifikation-Etikett (RFID-Etikett) umfasst, das in selektiver Kommunikation mit dem RFID-Sensor steht.

6. Kühlschrankgerät (100) nach Anspruch 5, wobei das RFID-Etikett ein passives RFID-Etikett ist.

7. Aktivfilter-Baugruppe (300), umfassend:
ein Gehäuse (302);
eine Einlassöffnung, die in dem Gehäuse (302) definiert ist;
eine Auslassöffnung, die in dem Gehäuse (302) definiert ist;
ein Filtermedium (319), das entfernbar innerhalb des Gehäuses (302) in Fluid-Verbindung zwischen der Einlassöffnung und der Auslassöffnung positioniert ist;
ein Feld-Etikett (342), das fest an dem Filtermedium (319) angebracht ist;
einen Feldsensor (344), der an dem Gehäuse (302) angebracht ist, um das Feld-Etikett (342) zu erkennen;
ein Gebläse (316), das innerhalb des Gehäuses (302) angeordnet ist und dazu betriebsfähig ist, aktiv Luft durch die Einlassöffnung und Auslassöffnung zu strömen; und
eine Steuereinheit (250) in betriebsfähiger Kommunikation mit dem Feldsensor (344), wobei die Steuereinheit (250) dazu eingerichtet ist, einen Filtriervorgang einzuleiten, wobei der Filtriervorgang umfasst:
Empfangen eines Erkennungssignals von dem Feldsensor (344) basierend auf der Erkennung des Filtermediums (319) innerhalb des Gehäuses (302) und
Führen einer Luftfilterung durch die aktive Luft-Baugruppe als Reaktion auf das Empfangen des Erkennungssignals, wobei das Führen der Luftfilterung selektives Drehen des Gebläses (316) umfasst, wobei der Feldsensor (344) einen Magnetfeldsensor (344) umfasst und wobei das Feld-Etikett (342) einen an dem Filtermedium (319) fest angebrachten Dauermagnet in selektiver Kommunikation mit dem Magnetfeldsensor (344) umfasst.

8. Aktivfilter-Baugruppe (300) nach Anspruch 7, wobei das Filtermedium (319) ein Fotokatalysator-Filtermedium umfasst, wobei
die Aktivfilter-Baugruppe (300) ferner eine Lichtquelle (324) umfasst, die innerhalb des Gehäuses (302) montiert ist.

9. Aktivfilter-Baugruppe (300) nach Anspruch 8, ferner umfassend eine Tür (128), die an dem Gehäuse (302) angebracht ist, wobei die Lichtquelle (324) auf die Tür (128) gerichtet ist.

10. Aktivfilter-Baugruppe (300) nach Anspruch 7, wobei das Feld-Etikett (342) innerhalb des Filtermediums positioniert ist.

11. Aktivfilter-Baugruppe (300) nach Anspruch 7, wobei die Aktivfilter-Baugruppe (300) ferner einen Mediumrahmen (330) umfasst, der zur Stützung des Filtermediums (319) fest an diesem angebracht ist, und wobei das Feld-Etikett (342) auf dem Mediumrahmen (330) positioniert ist.

12. Aktivfilter-Baugruppe (300) nach Anspruch 7, wobei der Feldsensor (344) einen Radiofrequenzidentifikation-Sensor (RFID-Sensor) umfasst und wobei das Feld-Etikett (342) ein fest an dem Filtermedium (319) angebrachtes Radiofrequenzidentifikation-Etikett (RFID-Etikett) umfasst, das in selektiver Kommunikation mit dem RFID-Sensor steht.

13. Aktivfilter-Baugruppe (300) nach Anspruch 12, wobei das RFID-Etikett ein passives RFID-Etikett ist.

## Revendications

1. Un appareil frigorifique (100) comportant:
une armoire (10) définissant une chambre réfrigérée (122);
une porte (128) pour accéder à la chambre réfrigérée (122);
un ensemble filtre actif (300) monté à l'intérieur de l'armoire (110) en communication fluidique avec la chambre réfrigérée (122),
l'ensemble filtre actif (300) comprenant un boîtier (302), un support filtrant (319) placé de manière amovible à l'intérieur du boîtier (302),
**caractérisé par** l'ensemble filtre actif (300) comprenant en outre une balise de champ (342) fixée au niveau du support filtrant (319), et un capteur de champ (344) fixé au boîtier (302) pour détecter la balise de champ (342); et
un contrôleur (250) en communication opérationnelle avec l'ensemble filtre actif (300), le contrôleur (250) étant configuré pour lancer une opération de filtration, l'opération de filtration comprenant
la réception d'un signal de détection à partir du capteur de champs (344) basé sur la détection du support filtrant (319) à l'intérieur du boîtier (302), et
l'orientation de la filtration de l'air à travers l'ensemble à air actif en réponse à la réception du signal de détection, dans lequel le capteur de champ (344) comprend un capteur de champ magnétique (344), et dans lequel la balise de champ (342) comprend un aimant permanent fixé au niveau du support filtrant (319) en communication sélective avec le capteur de champ magnétique (344).

2. L'appareil frigorifique (100) selon la revendication 1, dans lequel le support filtrant (319) comprend un milieu filtrant par photocatalyseur, dans lequel
l'ensemble filtre actif (300) comprend en outre une source lumineuse (324) montée à l'intérieur du boîtier (302), dans lequel
la source lumineuse (324) est dirigée vers la chambre réfrigérée (122).

3. L'appareil frigorifique (100) selon la revendication 1, dans lequel la balise de champ (342) est positionnée à l'intérieur du milieu filtrant.

4. L'appareil frigorifique (100) selon la revendication 1, dans lequel l'ensemble filtre actif (300) comprend en outre un cadre de support (330) fixé au niveau du support filtrant (319) en soutien à celui-ci, et dans lequel la balise de champ (342) est positionnée sur le cadre de support (330).

5. L'appareil frigorifique (100) selon la revendication 1, dans lequel le capteur de champ (344) comprend un capteur d'identification par radiofréquence (IDRF), et dans lequel la balise de champ (342) comprend une balise d'identification par radiofréquence (IDRF) fixée au niveau du support filtrant (319) en communication sélective avec le capteur d'IDRF.

6. L'appareil frigorifique (100) selon la revendication 5, dans lequel la balise d'IDRF est une balise d'IDRF passive.

7. Un ensemble filtre actif (300) comprenant:
un boîtier (302);
une ouverture d'entrée définie dans le boîtier (302);
une ouverture de sortie définie dans le boîtier (302);
un support filtrant (319) placé de manière amovible à l'intérieur du boîtier (302) en communication fluidique entre l'ouverture d'entrée et l'ouverture de sortie;
une balise de champ (342) fixée au niveau du support filtrant (319);
un capteur de champ (344) fixé au boîtier (302) pour détecter la balise de champ (342);
un ventilateur (316) disposé à l'intérieur du boîtier (302) et pouvant fonctionner activement afin de faire circuler l'air à travers l'ouverture d'entrée et l'ouverture de sortie; et
un contrôleur (250) en communication opérationnelle avec le capteur de champ (344), le contrôleur (250) étant configuré pour lancer une opération de filtration, l'opération de filtration comprenant
la réception d'un signal de détection du capteur de terrain (344) basé sur la détection du milieu filtrant (319) à l'intérieur du boîtier (302), et
l'orientation de la filtration de l'air à travers l'ensemble à air actif en réponse à la réception du signal de détection, la filtration d'air dirigée comprenant la rotation sélective du ventilateur (316), dans lequel le capteur de champ (344) comprend un capteur de champ magnétique (344), et dans lequel la balise de champ (342) comprend un aimant permanent fixé au niveau du support filtrant (319) en communication sélective avec le capteur de champ magnétique (344).

8. L'ensemble filtrant actif (300) selon la revendication 7, dans lequel le support filtrant (319) comprend un milieu filtrant par photocatalyseur, dans lequel l'ensemble filtre actif (300) comprend en outre une source lumineuse (324) montée à l'intérieur du boîtier (302).

9. L'ensemble filtre actif (300) selon la revendication 8, comprenant en outre une porte (128) fixée au niveau du boîtier (302), dans lequel la source lumineuse (324) est dirigée vers la porte (128).

10. L'ensemble filtre actif (300) selon la revendication 7, dans lequel la balise de champ (342) est positionné dans le milieu de filtration.

11. L'ensemble filtre actif (300) selon la revendication 7, dans lequel l'ensemble filtre actif (300) comprend en outre un cadre de support (330) fixé au niveau du support filtrant (319) en soutien à celui-ci, et dans lequel la balise de champ (342) est positionnée sur le cadre de support (330).

12. L'ensemble filtre actif (300) selon la revendication 7, dans lequel le capteur de champ (344) comprend un capteur d'identification par radiofréquence (IDRF), et dans lequel le champ de balise (342) comprend une balise d'identification par radiofréquence (IDRF)) fixée au niveau du support filtrant (319) en communication sélective avec le capteur d'IDRF.

13. L'ensemble filtre actif (300) selon la revendication 12, dans lequel la balise d'IDRF est une balise d'IDRF passive.
